(19) Europäisches Patentamt
European Patent Office
Office européen des brevets

(11) Veröffentlichungsnummer: **0 307 489**
**A1**

(12) # EUROPÄISCHE PATENTANMELDUNG

(21) Anmeldenummer: **87113482.1**

(51) Int. Cl.[4]: **A61K 31/47**

(22) Anmeldetag: **15.09.87**

Ein Antrag gemäss Regel 88 EPÜ auf Hinzufügung von Text zu Anspruch 2 liegt vor. Über diesen Antrag wird im Laufe des Verfahrens von der Prüfungsabteilung eine Entscheidung getroffen werden (Richtlinien für die Prüfung im EPA, A-V, 2.2).

(43) Veröffentlichungstag der Anmeldung:
**22.03.89 Patentblatt 89/12**

(84) Benannte Vertragsstaaten:
**BE CH DE FR GB LI SE**

(71) Anmelder: **S O "PHARMACHIM"**
**Iliensko Chaussée 16**
**Sofia(BG)**

(72) Erfinder: **Markov, Marko Tzonev**
**Bl. 435, Komplex Mladost IV**
**Sofia(BG)**
Erfinder: **Ivanov, Tchavdar Borissov**
**Bl. 46-4 Komplex Mladost I**
**Sofia(BG)**
Erfinder: **Jelyazkov, Deitcho Georgiev**
**69, Blvd. G. Dimitrov**
**Varna(BG)**
Erfinder: **Mondeschka, Dijana Minkova**
**125, Rakovska St.**
**Sofia(BG)**
Erfinder: **Berova, Nikolina Dimitrova**
**Bl. 194-3, Komplex Krasno Sela**
**Sofia(BG)**
Erfinder: **Rakovska, Rositza Stefanova**
**Bl. 37, Blvd. Vaptzarov**
**Sofia(BG)**
Erfinder: **Todorova, Maria Georgieva**
**18, Blvd. Lenin**
**Sofia(BG)**
Erfinder: **Popova, Dijana Dimitrova**
**3, Kozlodui St.**
**Sofia(BG)**
Erfinder: **Slavova, Emilija Danailova**
**Bl. 13-1 Zona B-5**
**Sofia(BG)**
Erfinder: **Zikalova, Tatjana Stoikova**
**31, P. Enev St.**
**Sofia(BG)**
Erfinder: **Marinova, Viola Marinova**
**Lozenska Planina St.**
**Sofia(BG)**

EP 0 307 489 A1

Erfinder: **Ovtcharov, Radi Georgiev**
**33, B. Kiro St.**
**Sofia(BG)**
Erfinder: **Uzunov, Petko Dimitrov**
**31 Januari Str. 3,**
**Sofia(BG)**
Erfinder: **Nissimov, Jossif Nissim**
**37, Sofronii St.**
**Sofia(BG)**
Erfinder: **Gentcheva, Dobrinka Gentcheva**
**Bl. 114A-4, S. Andonov St.**
**Sofia(BG)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **Antiglaukommittel.**

(57) Das Antiglaukommittel enthält als aktiven Stoff Hexahydrodibenzo(a,f)-chinoliziniumverbindungen in Form von Razematen oder optisch aktiven Formen mit der allgemeinen Formel I sowie ihre physiologisch verträglichen Salze als Gemisch mit ophthalmologischen Hilfstoffen. In der Formel I

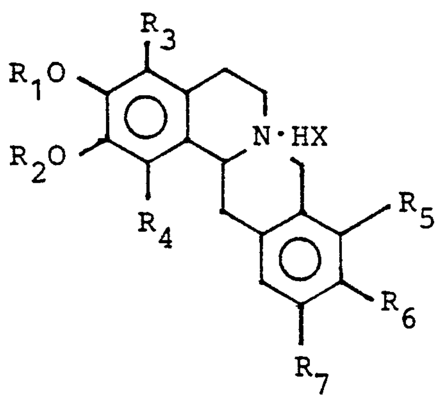

stehen $R_1$ und $R_2$ für H, $CH_3$ oder $CH_3CO$, $R_3$ und $R_4$ für H oder OH, $R_5$, $R_6$ und $R_7$ für H, OH oder $CH_3$ und X für ein Halogenatom.

Das Antiglaukommittel enthält insbesondere als aktiven Stoff s-(-)-2,3-dihydroxy-9,10,11-trimetoxy-5,8,13,13a-tetrahydro-6H-dibenzo(a,f)-Chinoliziniumhydrochlorid sowie passende pharmazeutische Träger.

## ANTIGLAUKOMMITTEL

Die Erfindung betrifft ein Antiglaukommittel.

Die am häufigsten gebrauchten Präparate für die Glaukombehandlung sind bekanntlich Pilocarpin, Epinephrin und Thymolol. Diese Präparate haben zwar eine gute therapeutische Wirkung, jedoch auch viele Nebenwirkungen. So verursacht zum Beispiel das Pilocarpin eine Myose, Spasmen der augennahen Muskeln, eine Hyperämie der Konjunktiva und allergische Reaktionen im Auge (Okeda, A. et al. - Duodecim 95, 1979, 11-15, Schiffer, H. - Merck Sharp Dohme Intern., 1978, 49-52). Das Epinephrin hat eine kurze und schwache Wirkung auf den Augeninnendruck und verursacht Tachykardie, Mydriase und Hyperämine (Katz, I. - Ann.Ophthalmol., 10, 1978, 847-850; Leydhecker, W. -Klin.Monatsbl. Augenheilkd., 171, 1977, 538-547). Das Thymolol zeigt bei mehrfacher lokaler Anwendung eine Reihe von Nebenwirkungen, nämlich Bradykardie, Hypotension, Bronchospasmen, besonders bei Asthma- und Bronchitiskranken und Symptome seitens des zentralen Nervensystems, wie Depression, Schläfrigkeit,. die seine Anwendung be beschränken (Arrata,M. - Ocular pharmacology of thymolol drops. London, Acad.Press, 1980; Katz, I. - Invest. Ophthalmol.Vis.Sci., 15, 1976, 489-492; Kosman,M. - JAMA 241, 1979, 2301-2303; Zimmerman, T. - Ophthalmol.Vis.Sci., 16, 1977, 687-688).

Der Erfindung liegt die Aufgabe zugrunde, ein Antiglaukommittel bereitzustellen, das den arteriellen Druck und die chronotropische Herzfunktion (untersucht an normotensiven Tieren) nicht wesentlich verändert, bei lokaler Anwendung im Auge keine entzündende Wirkung ausübt und keine allergischen Reaktionen verursacht.

Diese Aufgabe wird erfindungsgemäß durch die Verwendung eines physiologisch verträglichen Salzes von Hexahydrodibenzo (a,f) Chinoliziniumverbindungen in Form von Razematen oder optisch aktiven Formen mit der allgemeinen Formel I

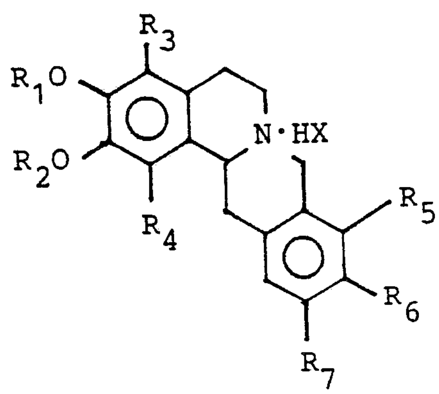

als Antiglaukommittel erreicht, wobei $R_1$ und $R_2$ für H, $CH_3$ oder $CH_3CO$, $R_3$ und $R_4$ für H oder OH, $R_5$, $R_6$ und $R_7$ für H, OH oder $CH_3O$ und x für ein Halogenatom, vorzugsweise Cl, stehen.

Es ist zwar bekannt, daß einige Hexadihydrobenzo(a,f)chinoliziniumverbindungen synthetischen oder natürlichen Ursprungs eine kurzfristige hypotensive Wirkung (Shimamoto,K. - Nippon lakugaku Zasshi, 53, 1957, 75-80), eine Anticholinesteraseaktivität (Berezhinskaja, V., E.Aleshinskaja - Pharmakol. i Toksikol., 31, 1986, p.196-199) oder eine depressive Wirkung auf das zentrale Nervensystem (Jamahara,J., T. Konoshima - Chem. Pharm.Bull., 24 (8), 1976), 1902-1912) ausüben. Weiterhin können sie eine gallenerregende (Velludo, C., T.Coina, I.Ticsa - Lucrarile presentate conf.Natl. Farm., Bucharest, 1958, 351-354), antibakterielle (Amin, A., T.Subbajah - Can.J.Microbiol., 15 (9), 1969, 1067-1076), entzündungshemmende und Magengeschwüre unterbindende Wirkung (Ikram, M. - Planta Med., 28 (4), 1975, 353-358) ausüben. Es gibt aber keine Angaben über eine Antiglaukomaktivität der Hexahydrodibenzo(a,f)chinoliziniumverbindungen.

Die höchste Antiglaukomaktivität in dieser Reihe von Verbindungen zeigt s-(-)-2,3-dihydroxy-9,10,11-trimetoxy-5,8,13,13a-tetrahydro-6H-dibenzo(a,f)Chinoliziniumhydrochlorid, im folgenden mit Verbindung Ia bezeichnet, deren Herstellung und Eigenschaften in BG-A-32353 beschrieben sind.

Die erfindungsgemäßen Verbindungen mit der allgemeinen Formel I haben folgende Vorteile: Sie verursachen keine Bronchospasmen und Bradykardie bei systematischem lokalen Anwenden im Vergleich mit Thymolol; sie besitzen eine schwach ausgedrückte und eine kurzfristige hypotensive Wirkung; sie üben keine entzündende Wirkung bei lokaler Anwendung im Auge aus und sie verursachen keine Myose, Hyperämie oder allergische Reaktionen bei systematischer lokaler Anwendung im Auge.

Die Verbindungen mit der allgemeinen Formel I können für die Glaukombehandlung in der Form von ophthalmologischen Lösungen, welche Lösungen der aktiven Komponente und der zusätzlichen pharmazeutischen Substanzen in Wasser sind, verwendet werden. Die ophthalmologischen Lösungen nach der Erfindung enthalten 0,25 bis 0,5 % der Verbindung Ia, welche die höchste Aktivität aufweist. Die ophthalmologischen Lösungen sind steril und können antimikrobiale Wirkstoffe, wie Benzalkoniumchlorid, Phenylquecksilbernitrat oder Thymerosal enthalten. Als Antioxydantin können Thioharnstoff, Thioglyzerin, Ascorbinsäure oder Natriummetabisulfit verwendet werden. Die ophthalmologischen Lössungen werden mit Hilfe von bekannten technologischen Verfahren zubereitet und in therapeutischen Dosen von 2 bis 3 Tropfen zwei bis vier Mal täglich angewendet.

Anhand von Beispielen wird die Erfindung unter Bezugnahme auf 3 Figuren näher erläutert. Es zeigt:

Fig. 1 in einem Diagramm einen Vergleich der Wirkungen der Verbindung Ia und von Thymolol auf den Augeninnendruck von nicht anasthesierten Kaninchen nach lokaler Anwendung gemessen mit dem Tonometer nach Schiotz,

Fig. 2 in einem Diagramm den Vergleich von Fig. 1, wobei die Werte mit dem Tonometer nach Maklalov gemessen sind und

Fig. 3 in einem Diagramm die chronotropischen Auswirkungen der Verbindung Ia und von Isoprenalin an nicht anästhetisierten Kaninchen.

Beispiel 1

s-(-)-2,3-dihydroxy-9,10,11-trimetoxy-5,8,13,13a-tetrahydro-6H-dibenzo(a,f)chinoliziniumhydrochlorid 50 g
Natriummetabisulfit 4 g
Dinatriumsalz der Ethylendiamintetraessigsäure 2 g
Benzalkoniumchlorid 1 g
Glyzerin 470 g
Wasser bis 10 l.

Das frisch destillierte Wasser wird 20 bis 30 min gekocht und danach mittels Durchblasens mit reinem Stickstoff gesättigt. Nachher wird es auf 40 bis 50° C abgekühlt. In ca. 8 l des gesättigten Wassers werden der Reihe nach Natriummetasulfit, Dinatriumsalz der Ethylendiamintetraessigsäure, die Verbindung Ia, Glyzerin und Benzalkoimchlorid bei ununterbrochener Sättigung mit Stickstoff gelöst. Die fertige Lösung wird durch einen Filter mit einer Membrane mit Öffnungsgröße von 0,22 µm filtriert. Die Abfüllung in Ampullen erfolgt bei doppelter Begasung mit Stickstoff.

Beispiel 2 Untersuchung des Ophthalmotonus

Die Spannung des Augapfels wird bei nicht narkosierten und normotensiven Kaninchen untersucht. Dabei wird in einer ersten Untersuchungsreihe der Augeninnendruck mit einem Tonometer nach Schiotz gemessen und in mm/Hg nach der Skala von Leydhecker berechnet (Fig. 1). In einer zweiten Untersuchungsreihe wird der Augeninnendruck mit einem Tonometer nach Maklakov gemessen (Fig. 2).

Die Vergleichsuntersuchungen, die mit der Verbindung Ia und Thymolol bei lokaler Anwendung im Auge in Form einer 0,5 %igen wässrigen Lösung durchgeführt wurden, zeigen, daß die Verbindung Ia eine statistisch bedeutsame Verminderung des Innendrucks, gemessen mit dem Tonometer nach Schiotz, mit einem Maximum nach 60 bis 120 min verursacht, wobei ein bedeutsamer Unterschied ihrer Wirkung im Vergleich zu Thymolol nicht festgestellt wurde, was im einzelnen aus Fig. 1 ersichtlich ist.

Ähnliche Ergebnisse ergeben sich, wenn die Messungen des Augeninnendrucks mit dem Tonometer nach Maklalov durchgeführt werden, was in Fig. 2 dargestellt ist. Weitere Vergleichsversuche, durchgeführt mit nicht narkosierten Ratten (Linie Vistar) zeigen, daß die Verbindung Ia im Unterschied zu Thymolol bei lokaler Anwendung keine statistisch bedeutsamen Veränderungen im Arterialdruck und in der Herzfrequenz verursacht, was sich im einzelnen aus Tabelle 1 ergibt.

## TABELLE 1

Vergleichsversuche über die Wirkung der Verbindung Ia und von Thymolol auf den Blutdruck und die Herzfrequenz bei lokaler Anwendung an nicht narkosierten Ratten

| SUBSTANZ | Anfangsdaten Blutdruck mm/Hg systol. | Anfangsdaten Blutdruck mm/Hg diastol. | Anfangsdaten Herz-Frequenz Schläge/min. | nach 20 min. Blutdruck mm/Hg systol. | nach 20 min. Blutdruck mm/Hg diastol | nach 20 min. Herz-Frequenz Schläge/min. | nach 60 min. Blutdruck mm/Hg systol. | nach 60 min. Blutdruck mm/Hg diastol | nach 60 min. Herz-Frequenz Schl./min. |
|---|---|---|---|---|---|---|---|---|---|
| 0,5%ige Lösung der Verbindung Ia | 182 ± 21,9 | 144 ± 19,7 | 384 ± 24,2 | 164 ± 18,7 (p>0,05) ↓ 9,9 % | 124 ± 17,8 (p>0,05) ↓13,9 % | 388 ± 22,2 (p>0,05) | 131 ± 11,2 (p>0,05) ↓28 % | 96 ± 12,9 (p>0,05) ↓33,3 % | 362 ± 15,3 (p>0,05) ↓5,7 % |
| 0,5%ige Lösung von Thymolol | 198 ± 7,3 | 168 ± 7,0 | 405 ± 17,9 | 184 ± 8,4 (p>0,05) ↓ 7 % | 155 ± 8,0 (p>0,05) ↓7,7 % | 339 ± * 20,2 (p<0,05) ↓16 % | 162 ±* 8,8 (p<0,05) ↓18 % | 135 ±* 9,0 (p<0,05) ↓20 % | 314 ± ** 9,7 (p<0,01) ↓22 % |

*p < 0,05
**p < 0,01 (n = 12)

Beispiel 3 Einfluß auf das Herz-Blutgefäß-System

Die Verbindung Ia vermindert bei intravenöser Anwendung in Dosen von 0,1; 0,5; 1; 2; 3 und 5 mg den Blutdruck von narkosierten Katzen und Kaninchen. Seine hypotensive Wirkung besonders bei niedrigen Dosen ist schwach ausgeprägt und kurzfristig. Bei einer Dosis von 0,25 mg/kg beträgt sie 22 % für eine Dauer von 10 min (Tabelle 2).

TABELLE 2

| Einfluß der Verbindung Ia auf den Blutdruck von Katzen | | |
|---|---|---|
| Dosis mg/kg | Hypotensive Wirkung % | Dauer min |
| 0,1 | 18 | 8 |
| 0,25 | 22 | 10 |
| 0,5 | 25 | 24 |
| 1 | 33 | 30 |
| 2 | 38 | 45 |
| 3 | 42 | über 60 |
| 5 | 52 | über 60 |

Eine mit einigen Mediatoren und anderen Versuchswirkstoffen durchgeführte Analyse zeigt, daß auf dem Hintergrund der Verbindung Ia (3 mg/kg) die Wirkung von Dopamin und Isoprenalin hinsichtlich des Blutdrucks sowie die pressorische Wirkung der Okklusion der Kopfschlagader vermindert wird, während sich die pressorische Wirkung des Noradrenalins erhöht. Letzteres ist wahrscheinlich mit der Stimulierung der vorsynaptischen beta-Rezeptoren, die die Aus scheidung des Mediators Noradrenalin erleichtern, verbunden (Tabelle 3). Die hypotensive Wirkung der Verbindung Ia wird wesentlich auf dem Hintergrund des Propranols - 1,5 mg/kg intravenös - unterdrückt.

Die durchgeführten Untersuchungen mit nicht narkosierten Kaninchen zeigen, daß die Verbindung Ia eine wesentlich schwächer ausgeprägte Wirkung und eine kurzfristigere positive chronotropische Wirkung im Vergleich mit Isoprenalin aufweist, weil sie keine statistisch bedeutsame Veränderungen im Chronotropismus des Herzens verursacht (Fig. 3). Im Unterschied zu Thymolol verändert die Verbindung Ia keineswegs die statistisch zuverlässige chronotropische Funktion des Herzens (Tabelle 4).

TABELLE 3

| Wechselwirkung der Verbindung Ia mit einigen Mediatoren und anderen Prüfstoffen hinsichtlich des Blutdrucks von Katzen | | | |
|---|---|---|---|
| Prüfstoff | Verbindung Ia mg/kg | Veränderungen des Blutdrucks % | |
| | | vorher | nachher |
| Dopamin, 20 mkg/kg | 2 | -16,6 | -8,3 |
| Dopamin, 40 mkg/kg | 2 | -23,0 | -4,2 |
| Isoprenalin, 1,5 mkg/kg | 3 | -30,5 | -8,8 |
| Noradrenalin, 3 mkg/kg | 1,5 | +21,0 | +46,0 |
| Noradrenalin, 3 mkg/kg | 3,0 | +17,0 | +33,0 |
| Histamin, 5 mkg/kg | 5 | -52,0 | -23,0 |
| Okklusion der Schlagader | 2 | | -40,0 |
| | 5 | | -53,0 |
| Propranolol, 1,5 mg/kg | 2 | -42,0 | -24,0 |

Beispiel 4 Untersuchung der lokalen Verträglichkeit

Die lokale Verträglichkeit der Verbindung Ia in Form der 0,5 %igen wässrigen Lösung wurde nach zwei Verfahren geprüft.

a) Verfahren nach Marzuli und Simon, nachgeprüft und eingeführt von Marzuli und Ruglas.

Für diesen Versuch wurden 12 weiße Kaninchen beiderlei Geschlechts mit Körpergewichten von 3200 bis 4000 g in zwei Gruppen von je 6 aufgeteilt. Die erste Gruppe wird mit der 0,5 %igen Lösung der Verbindung Ia behandelt, während die zweite nur mit dem Lösungsmittel behandelt wird. Dabei werden in den Konjunktivalsack des einen Auges 0,1 ml der Probe getropft, während das andere Auge als Kontrolle dient. Die lokale Augenreaktion wird in der 1., 24., 48. und 72. Stunde sowie am 7. Tag der Behandlung bestimmt. Es wird das von den Autoren angenommene standardisierte Punktsystem verwendet, nämlich für die Hornhaut 0 bis 4, für die Iris 0 bis 2, für die Bindehaut 0 bis 3 und für Hyperämie 0 bis 4. Die Beobachtung der Dynamik beider Gruppen ergibt keine Merkmale von lokaler Reizung und für alle drei Parameter Hornhaut, Iris und Konjunktiva. Die erhaltenen Ergebnisse zeigen, daß die Verbindung Ia eine gute Augenverträglichkeit aufweist und bei ihrer Anwendung in der Praxis keine Gefahr besteht, daß Entzündungen des Lidrandes oder der Bindehaut oder toxische Schädigungen der Hornhaut sowie des ganzen Auges auftreten.

b) Verfahren nach Mac Donald

Die Versuche werden mit 12 weißen Kaninchen bei gleicher Anzahl männlicher und weiblicher Kaninchen mit einem Mittelgewicht von 3700 g durchgeführt, die in zwei Gruppen zu je 6 aufgeteilt sind. Die erste Gruppe wird mit der 0,5 %igen Lösung der Verbindung Ia, die zweite nur mit dem Lösungsmittel behandelt. Dabei werden in den Konjunktivalsack des einen Auges 0,05 ml der zu untersuchenden Probe drei mal täglich 5 Tage lang getropft. Die lokale Augenreaktion wird jeden Tag vor der nachfolgenden Behandlung an der Hornhaut, Iris und Konjunktiva bestimmt und danach mit dem nicht behandelten Kontrollauge für jedes Kaninchen verglichen.

Bei den dreifachen Beobachtungen während der fünf Tage der Behandlung wurden keine Veränderungen der drei untersuchten Parameter festgestellt.

Tabelle 4

Chronotropische Wirkung der Verbindung Ia und von Timolol bei lokaler Anwendung auf nicht narkosierte Kaninchen

| Substanz | Herzfrequenz in Schlägen pro Minute | | | | | | |
|---|---|---|---|---|---|---|---|
| | Beginn | 5 min | 15 min | 30 min | 60 min | 120 min | 180 min |
| Verbindung Ia in 0,5%iger Lösung | 221,2 $\pm$16,5 | 228,3 $\pm$17,4 ($p \geq 0,05$) | 227,3 $\pm$17,8 ($p \geq 0,05$) | 217,8 $\pm$15,6 ($p \geq 0,05$) | 219,5 $\pm$16,0 ($p \geq 0,05$) | 221,2 $\pm$16,5 ($p \geq 0,05$) | 221,2 $\pm$16,5 ($p \geq 0,05$) |
| Thymolol in 0,5 %iger Lösung | 247,0 $\pm$6,4 | * 215,6 $\pm$8,4 ($p < 0,05$) | ** 204,0 $\pm$6,6 ($p < 0,01$) | ** 195,2 $\pm$5,6 ($p < 0,01$) | ** 196,6 $\pm$7,0 ($p < 0,01$) | ** 203,0 $\pm$6,8 ($p \leq 0,01$) | * 207,5 $\pm$6,1 ($p \leq 0,05$) |

* $p < 0,05$
** $p < 0,01$ ($n = 12$)

## Ansprüche

1. Antiglaukommittel, dadurch **gekennzeichnet,** daß es als aktiver Stoff Hexahydrodibenzo(a,f)-chinoliziniumverbindungen in Form von Razematen oder optisch aktiven Formen mit einer allgemeinen Formel I sowie ihre physiologisch verträglichen Salze als Gemisch mit ophthalmologischen Hilfsstoffen enthält, wobei in der Formel I

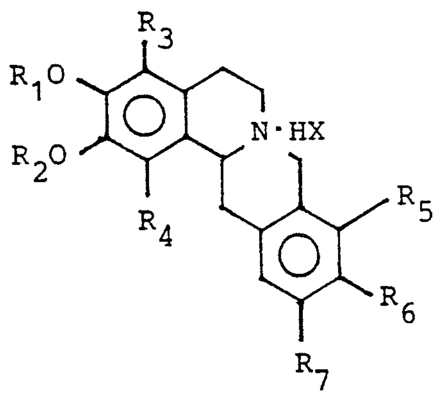

$R_1$ und $R_2$ für H, $CH_3$ oder $CH_3CO$, $R_3$ und $R_4$ für H oder OH, $R_5$, $R_6$ und $R_7$ für H, OH oder $CH_3O$ und X für ein Halogenatom stehen.

2. Antiglaukommittel nach Anspruch 1, dadurch **gekennzeichnet,** daß es als aktiven Stoff s-(-)-(a,f)-Chinoliziniumhydrochlorid ($R_1 = R_2 = R_3 = R_4 = H$; $R_5 = R_6 = R_7 = CH_3O$) sowie passende pharmazeutische Träger enthält.

3. Antiglaukommittel nach Anspruch 1, **gekennzeichnet** durch die Verwendung in Form von 0,5 %igen opthalmologischen Lösungen.

EPAA-35519.1

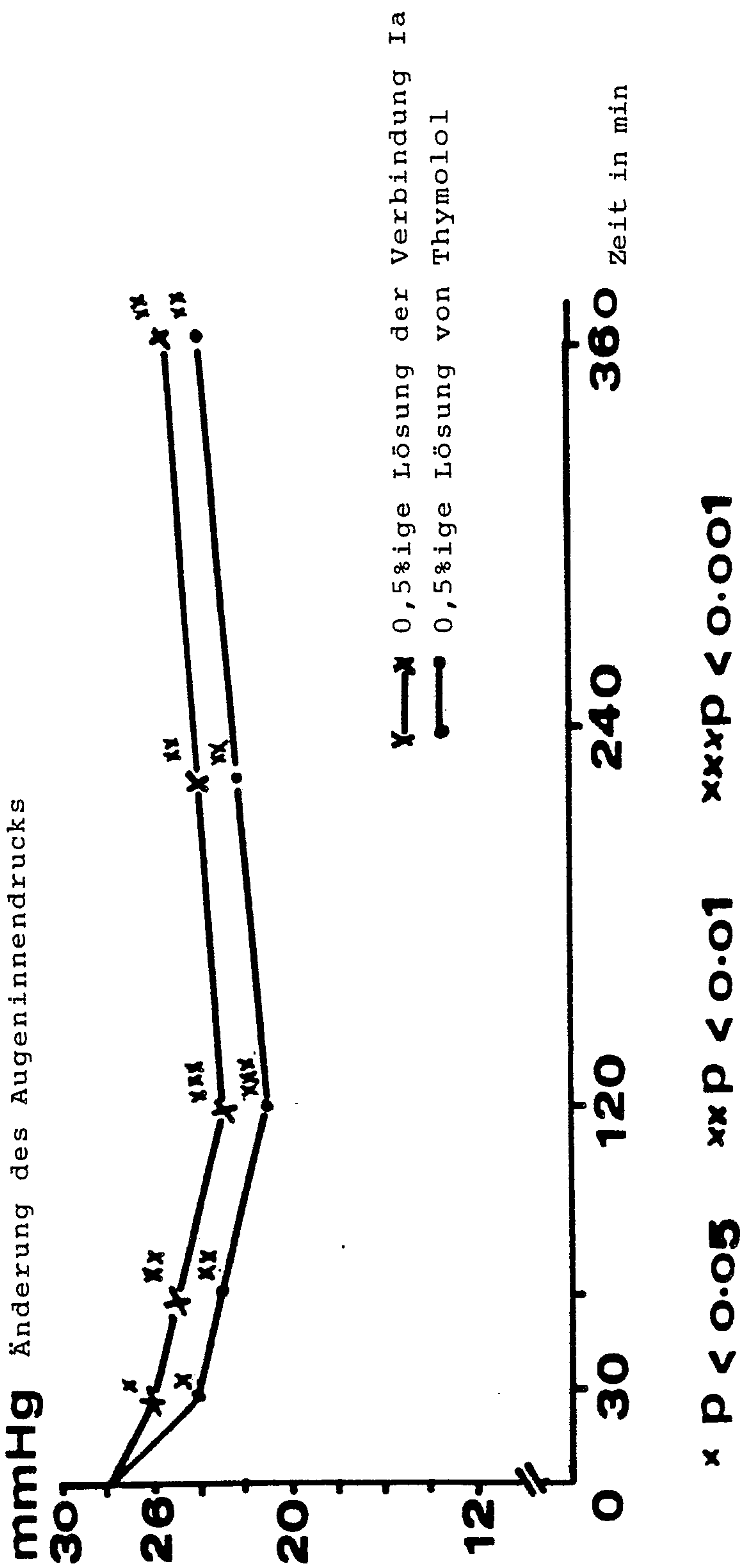


Fig. 1

EPAA-35519.1

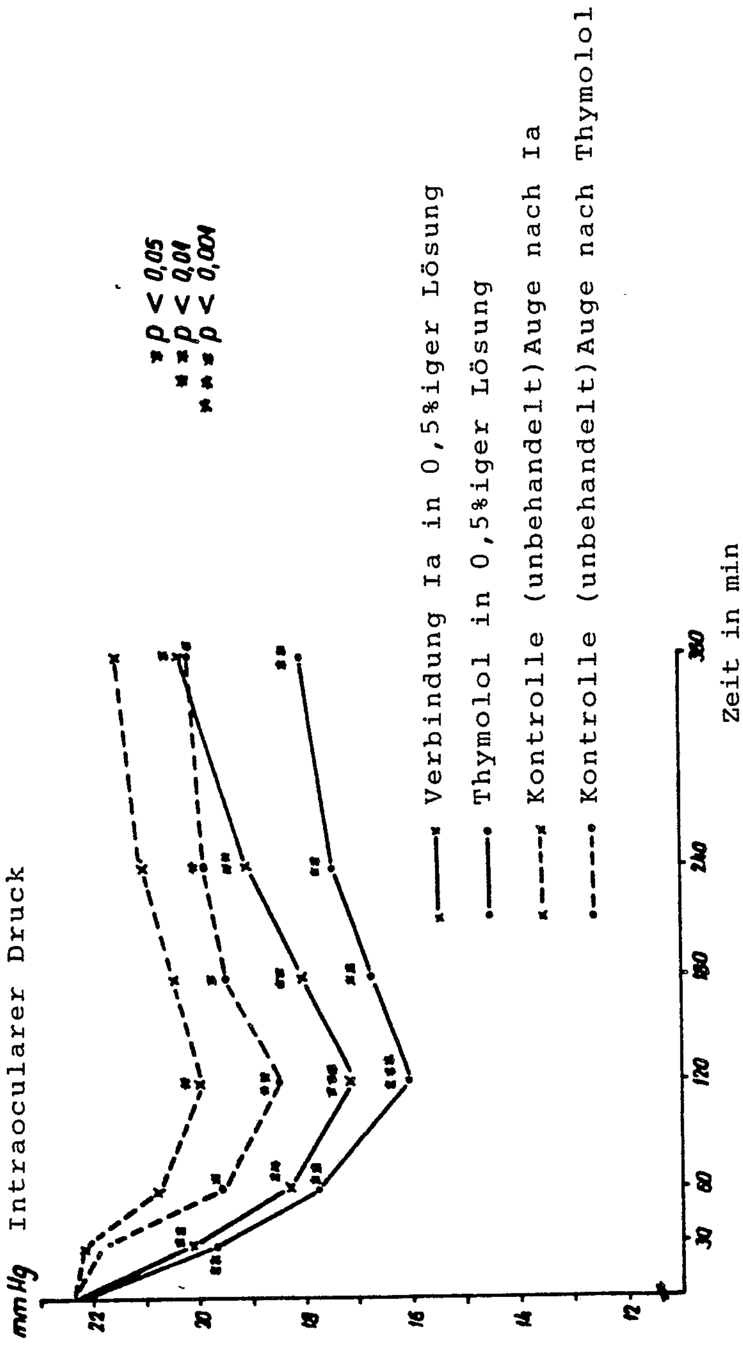


Fig. 2

EPAA-35519.1

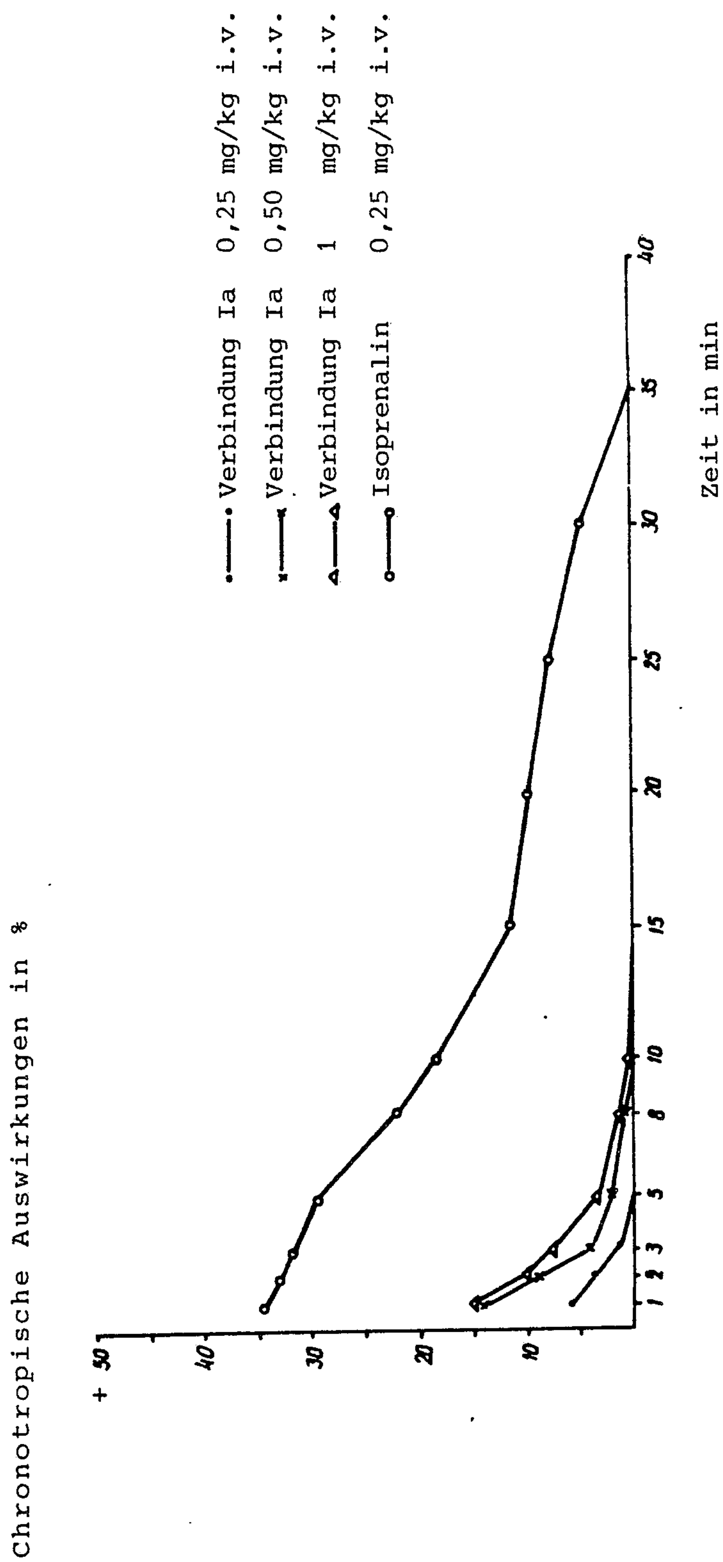


Fig. 3

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 87 11 3482

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CHEMICAL ABSTRACTS, Band 99, Nr. 7, 15. August 1983, Seite 12, Zusammenfassung 47506b Columbus, Ohio, US; D.A. WILLIAMS: "Identification of a tetrahydroprotoberberine as a metabolite of trimetoquinol in the rat", & BIOCHEM.PHARMACOL. 1983, 32(8), 1447-8<br>* Zusammenfassung *<br>-- | 1-3 | A 61 K 31/47 |
| Y | EUR.J.MED.CHEM. - CHIM.THER.1986, Band 21, Nr. 1, Seiten 45-48 I. CHAVDAR et al.: "Synthesis and antigluacomic activity of N-carbamoyl derivatives of trimethoquinol"<br>* Zusammenfassung; Figur 2 *<br>-- | 1-3 | |
| A | CHEMICAL ABSTRACTS, Band 83, Nr. 22, 1. Dezember 1975, Seite 495, Zusammenfassung 193577p Columbus, Ohio, US; & JP - A - 75 76 098 (JAPAN | | RECHERCHIERTE SACHGEBIETE (Int. Cl.4)<br>A 61 K 31/00 |

-2-

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1,3
Unvollständig recherchierte Patentansprüche: 2
Nicht recherchierte Patentansprüche:
Grund für die Beschränkung der Recherche:
Anspruch 2: Name nicht vollständig

| Recherchenort | Abschlußdatum der Recherche | Prüfer |
|---|---|---|
| DEN HAAG | 16.05.1988 | ISERT |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Europäisches Patentamt

**EUROPÄISCHER TEILRECHERCHENBERICHT**

Nummer der Anmeldung
EP 87 11 3482
-2-

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| | CHEMIPHA CO.LTD) 21-06-1975<br>* Zusammenfassung *<br>-- | 1 | |
| A | CHEMICAL ABSTRACTS, Band 85, Nr. 1, 5. Juli 1976, Seite 481, Zusammenfassung 5936z Columbus, Ohio, US; & JP - A - 75 129 600 (JAPAN CHEMIPHA CO.LTD) 13-10-1975<br>* Zusammenfassung *<br>-- | 1 | |
| A | CHEMICAL ABSTRACTS, Band 87, Nr. 16, 17. Oktober 1977, Seite 327, Zusammenfassung 141272y Columbus, Ohio, US; & JP - A - 77 64 417 (JAPAN CHEMIPHA CO.LTD) 25-11-1975<br>* Zusammenfassung *<br>-------------------- | 1 | RECHERCHIERTE SACHGEBIETE (Int. Cl.4) |